# EUROPEAN PATENT SPECIFICATION

(11) **EP 0 493 137 B1**
(45) Date of publication and mention of the grant of the patent: **23.09.1998**
(21) Application number: 91312089.5
(22) Date of filing: 30.12.1991
(51) Int. Cl.: C07K 14/00, H01B 1/20, H01B 1/22, H01B 1/24, H01B 3/18

(54) **A method for forming ultra-fine structures**
Verfahren zur Bildung ultrafeiner Strukturen
Procédé pour réaliser des structures ultrafines

(30) Priority: 28.12.1990 JP 409457/90; 08.11.1991 JP 293092/91
(43) Date of publication of application: 01.07.1992
(73) Proprietor: Research Development Corporation of Japan, Chiyoda-ku Tokyo (JP); MATSUSHITA ELECTRIC INDUSTRIAL CO., LTD, Kadoma-shi, Osaka 571 (JP)
(72) Inventor: Yamashita, Ichiro, Tsukuba-shi, Ibaragi (JP); Nanba, Keiichi, Tsukuba-shi, Ibaragi (JP)
(74) Representative: Pett, Christopher Phineas

(56) References cited:
- WO-A-88/08875
- NATURE. vol. 342, no. 6250, 7 December 1989, LONDON GB pages 648 - 654; K. NAMBA ET AL.: 'STRUCTURE OF THE CORE AND CENTRAL CHANNEL OF BACTERIAL FLAGELLA.

## Description

### [FIELD OF THE INVENTION]

This invention relates to a method for forming ultra fine-structures. More specifically, it relates to a method for forming ultra-fine structures which are useful for the connecting wires or connectors of micro electronic circuits as covered conductors, and which are also usable as ultra-micro functional materials.

### [PRIOR ART ]

Dramatic progress in electronics engineering during recent years, represented by the super computer, has made possible the high-speed processing of information which was heretofore impossible. This helps achieve the provision of more precise weather information, greater precision and higher degree in ultra LSI designs, and the development of innovative materials. To perform large-scale and highly precise calculations enough to be put to a practical application, however, computers operating at a speed several tens of times as high as that available at present are required, and for this purpose, at ultra-high speed operators and large-scale memories are necessary.

On the other hand, when we consider the signal transmission speed within a computer, even light, which travels the the fastest of all, can travel only 3 centimeters in 1/10 billion of a second aniticipated to be the speed of future ultra-high speed computers. To enhance the hardware performance of the computer, therefore, it is indispensable to miniaturize the system and construct it in higher density, as well as to make the circuit devices operate at higher speeds.

From such viewpoint, the electronics circuit presently available is being downsized from micron to submicron unit: such downsizing is expected to be further proceeding. In keeping up with this trend, downsizing is required of not only individual circuit devices, but also the wires used to connect the same.

Furthermore, a liquid crystal display (LCD) is, for example, becoming increasingly compact and lightweight, since liquid crystal is much thinner and lighter in weight than its similar devices, and very small voltages at which it is operated and driven are required.

Nevertheless, when we seek to further downsize the LCD, it becomes difficult to maintain the qualities of the screen (e.g., contrast and visibility) to high level. When a character is displayed on an LCD screen, such as LCD TV receiver, each LCD is normally driven by a method known as multi-matrix electrode system. In order to increases the display capacity by N times by this method, the driving circuit and terminal connections N times as many are required.

For this reason, to achieve the downsized system and increased display capacity, it becomes indispensable to make LCD driving electrode structure and electronics circuits more refined and finer, and also to downsize the connectors connecting therebetween to a finer size.

It is exceedingly difficult, however, to make the present microelectronics circuits much finer and more integrated. For instance, the wires of circuits, such as deposited metallic membrane used in semiconductor chips, are not insulated ; hence this uncovered wire not only requires the utmost care in handling, but also is essentially unsuited to three-dimensional wiring. For connecting liquid crystal devices and driving electronics circuits, pin connectors, elastic connectors or flexible connectors, for instance, are employed. These connectors are provided with only dozens of covered conductors per 1 mm, and even with semiconductor technologies, this conductor is in submicron units at the utmost. Under these circumstances, interest has been mounting in constructing biochip and biocircuits utilizing the intergration of proteins and functions of microorganisms.

Approaches to ultra-refinement and ultraintergration using these biodesign technologies are being noted in the medical and micromachine fields. W088/08875 describes a biochip sensor in which bioactive molecules capable of forming a complex with a ligand in aqueous solution are immobilised on a substratum; and binding of these molecules and ligands is detected electronically.

However, under the present circumstances, virtually no specific means to develop these biodesign technologies have been established. For instance, how living matter and organic molecules will be specifically used in a given location or structure remains to be known.

### [SUMMARY OF THE INVENTION]

The present invention has the objective of providing a method for forming ultra-fine structures which are useful as ultra-micro conductors which permit micro and high density wiring of micro electronics circuits, or ultra-fine functional materials necessary for the realization of micromachines.

The present invention provides a method for forming an ultra-fine structure comprising the steps of bonding atoms or molecules at least amino end of carboxyl end, or in the vicinity thereof, of flagellins constructing a bacterial flagellum, and polymerizing the flagellins.

This invention also provides a method for forming an ultra-fine structure in which the polymerized flagellin prepared by the foregoing method is oriented in a magnetic field.

### [BRIEF DESCRIPTION OF THE DRAWING]

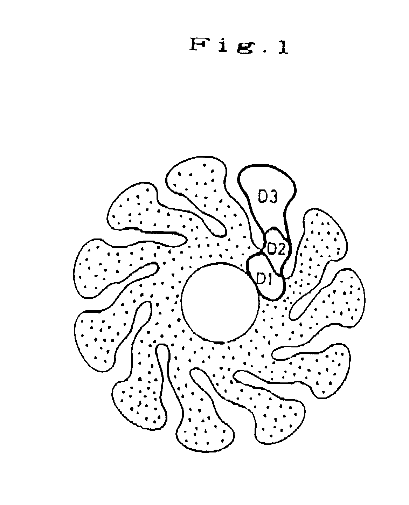

FIG. 1 indicates a front sectional view of the bacterial flagellum used to form ultra-fine structures according to the present invention. THe white-on-black portion in the figure illustrates the shape of flagellin, a subunit of the flagellum, and each of the domains thereof (D1, D2 and D3).

### [DETAILED DESCRIPTION OF THE INVENTION]

On the following pages, detailed descriptions will be described on the principle and construction of the present invention.

The ultra-fine structure according to the present invention is featured by being prepared using the characteristics unique to a flagellin, a subunit of a bacterial flagellum, i.e., self-assembly, amino acid sequence characteristics.

Flagellum is a helical filament consisting of single kinds of proteins known as flagellin, possessing a 6-nm-diameter central hole passing therethrough. The whole diameter thereof is as fine as approx. 25nm, but the flagellum is not limited in dimensions in the lengthwise direction, normally extending several times (10 - 20µm) of its bacterial body (see Namba et al in Nature; 342; 648-654 (1989).

The inventors of the present invention has discovered that the flagellin consists of three domains (D1, D2, D3) as indicated in FIG. 1, and that the D1 domain, located in the central core part and forming a central Channel is involved in a self-assembling formation of the flagella. Correlations between each domain and amino acid sequences revealed that the D1 domain consists of the ends of a amino acid sequence of flagellin, that is to say, the amino end and the carboxyl end.

The present invention has been made on the basis of the aforestated very significant knowledge.

Flagellum is re-constructed by monomerizing the flagellins of the flagellum, chemically bonding atoms or molecules of various metals, semi-metals and non-metals to the amino or carboxyl end of the D1 domain through covalent bonding, hydrogen bonding, etc., then by polymerizing these flagellins in a self-assembling manner. As a result, the thus re-constructed lagellum has a band consisting of these atoms and molecules on the inside peripheral surface thereof. When using conductive metals as atoms, for instance, a conductive band is formed. This conductive band is completely covered with flagellins, insulators, and hence an covered conductive ultra-micro structure with scores of µm long and with a diameter of approx. 20 nm can be prepared. When photo-responsive molecules are used, ultra-fine structure which consist of photo-responsive functional band regions is formed.

Of the domains constituting flagellins, the amino acid sequence in D1 domain is virtually in common with those in comprehensive kinds of bacteria. Accordingly, flagellins of a wide variety of bacteria can be used in this invention. Since the flagellins of salmonella strains are readily available and excel in linearity, they are especially preferred for the present invention.

Then, descriptions will be given more specifically about a method for forming ultra-fine structures according to the present invention, together with examples.

### (1) Preparation of flagellines

Flagellins, a subunit of flagellum, can be synthesized by genetic manipulation, and also can be extracted and prepared from the flagellum of bacterias by the ordinary method. For instance, the cultured bacteria is subjected to shear force, which is created in a buffer liquid consisting of Tris 20 mM (pH 7.8) + Nacl 0.15M. Then the flagella are collected by centrifugal separation. Then, these flagella are heat-treated at 65°C for ten minutes, providing a flagellins as a simple substance of monomerized flagella.

### (2) Binding of atoms or molecules

Atomic or molecular compounds are caused to be bound to the amino end or carboxyl end of proteins which constitute the D1 domain of individual flagellin through a known chemical reaction.

In this case, there are no special limitations to the type of atoms or molecules. Metallic, semiconductor or non-metallic atoms, or photo-conductive, magnetic-conductive and other functional organic or inorganic compounds can be employed. These atoms or molecules can be chemically bonded to the amino end or carboxyl end through covalent bond or hydrogen bond.

For example, more specifically, metallic or semiconducting atoms or molecules may be bound to amino acid residues on the wall surface of the central hole of the flagellum, and the following bindings can be examplified:
- Hg (mercury) :: binding to Arg (arginine), Cys (cysteine) and the like.
- Ag (silver) :: binding to His (histidine).
- Sm (samarium) :: binding to Glu (glutamic acid).

Other Pt, Au, and Pd are adhered to amino acid residues.

Silicon and germanium can be conceived as semiconductors. They can be introduced while silicon is allowed to be covalently bonded with the sulfur in cysteine residue or methionine residue since it is difficult for them to be bound to amino acid as they are.

As for nonmetal, a polymer to be crosslinked with ultraviolet rays, for example, can be bound to the amino end or carboxyl end of the flagellin.

### (3) Reconstruction of flagellum

Ammonium sulfate is added to a flagellin solution in which each flagellin binds atom or molecule, causing the flagellin to be polymerized in a self-assemblying manner and flagella to be reconstructed.

In this case, the length of the flagella depends upon the density of flagellins in a buffer liquid and upon the density of ammonium sulfate to be added thereto. For instance, when 1M ammonium sulfate is added to 3 to 10 mg/ml flagellin solution, approx. 0.5 µm flagellum is formed in several minutes. However, if the ammonium sulfate is 0.5M and the flagellin is 1mg/ml or less, it takes more than 10 hours to form 10 to 100 µm flagellum. Accordingly, it is preferred to add 0.5 to 2M ammonium sulfate to 1 to 10 mg/ml flagellin solution.

Lastly, ultra-fine structures having the specified atoms or molecules continuously on the wall surface of the central hole of the flagellum is completed by using Tris 20 mM (pH 7.8) + Nacl 0.15M, Gly 0.2M (pH 8.0) and the like as buffer liquid.

If conductive metal is bound to the central hole of such ultra-fine structure, the flagellar protein becomes an insulator, and hence this structure becomes an ultra-fine covered electrical wire which conducts electric current in its center alone. Since this electrical wire is completely covered, it can be used in any shape as covered wire. Especially if flagellins over which metal is bound is introduced at given intervals during the polymerization, the resulting structure becomes wire having an electrode at given intervals. If this covered conductive ultra-fine structure is brought into direct contact with metal, tunnel curent flows, forming a Josephson device.

Moreover, the ultra-fine structures formed by the foregoing process can have the atoms or molecules bonded to the central hole thereof bonded or crosslinked with each other by adding thermal or optical energy thereto. For instance, a flagellum polymers to which nonmetal polymers are bound become spiral-shaped ultra-fine line structures by crosslinking each of the polymere. Hence, this can be used directly as ultra-fine structured functional materials for springs.

Ultra-fine line structures in which the atoms or molecules in the central holes are bonded or crosslinked with one another in this way can decompose or eliminate the flagellins constituting the surroundings thereof by means of heating. By doing this, it is possible to form further ultra-fine structures which consist only of continuous atoms or molecules.

Then, detailed descriptions will be given as to a method for forming another ultra-fine structures according to the present invention.

This method forms ultra-fine structures using the magnetic configuration of the flagellum, which the inventors of this invention found, together with the aforestated self-assembly and amino acid sequence characteristics thereof.

On the following descriptions, an example will be given as to a method for forming one-dimensional conductor in which ultra-fine covered conductors are arranged parallel in ultra-fine pitches on the substrate.

Each ultra-fine covered conductor uses flagella to whose inner wall surface conductive materials are bonded. For this flagellum, the one of salmonella, particularly the linear mutant is desired. These flagella are dissolved in several percents in Gly 0.2M (pH 8.0) + 10 mM ammonium sulfate, and this solution is titrated onto the substrate. If this substrate is located in a magnetic field so that the surface onto which the solution is titrated becomes parallel therewith, each flagellin to which conductive materials are bound is oriented in parallel with the direction of magnetic field as an axis, due to its magnetic configuration. Lastly, if the solution is allowed to dry, one-dimensional conductor of ultra-fine pitches is formed on the substrate. If it is allowed to dry, the operation is performed so that it will take the solution about one hour to become dried.

The one dimensional conductor thus formed has each conductor thereof covered with proteins. And hence, if the end is press-fit, an electronic junction with electrode terminals can be readily obtained, and for example, can be used as connectors of fine electronic circuits.

### [EFFECTS OF THE INVENTION]

As has been described in detail above, the present invention provides ultra-fine structures which utilize bacterial flagella, as one embodiment of the bio-design technology.

These ultra-fine structures are useful for the three-dimensional wiring or connectors of micro electronics circuit as covered micro conductors, as well as for micromachines as various functional materials.

## Claims

1. A method for forming an ultra-fine structure, which comprises binding atoms or molecules to at least the amino end or the carboxyl end of one or more flagellins, and assembling a number of said flagellins to form said structure.

2. A method according to claim 1, wherein the assembled flagellins are heated or irradiated to cause the atoms or molecules bound thereto to be bonded or crosslinked to one another.

3. A method according to claim 2, wherein the flagellins are eliminated from assembled flagellins in which the atoms or molecules bound thereto are bonded or crosslinked to one another in order to form an ultra-fine structure which consists only of continuous atoms or molecules.

4. A method as claimed in claim 1 which comprises causing the assembled flagellins to be oriented in one direction in a magnetic field.

5. A method according to any one of the preceding claims, wherein the bacterial flagellin is a sub-unit of a bacterial flagellum.

6. A method as claimed in claim 5 in which the flagellin is derived from a salmonella species.

7. A method as claimed in any of the preceding claims in which the atoms or molecules bound to said amino or carboxyl ends form an electrically conductive filament.

8. An ultrafine structure obtainable by the method of any one of claims 1 to 7.

9. Flagellin molecules having atoms or molecules attached to at least the amino end or the carboxyl end so that a flagellin-like structure is formed upon assembly.

10. An array of flagellin-like structures comprising flagellin molecules as claimed in claim 9.

## Patentansprüche

1. Ein Verfahren zur Bildung einer Ultrafeinstruktur, welches das Binden von Atomen oder Molekülen an wenigstens das Aminoende oder das Carboxylende von einem oder mehreren Flagellinen und das Zusammenfügen einer Reihe der Flagellinen, um die Struktur zu bilden, umfaßt.

2. Ein Verfahren gemäß Anspruch 1, bei dem die zusammengefügten Flagelline erwärmt oder bestrahlt werden, um zu bewirken, daß die daran gebundenen Atome oder Moleküle miteinander verbunden oder vernetzt werden.

3. Ein Verfahren gemäß Anspruch 2, bei dem die Flagelline von zusammengefügten Flagellinen, bei denen die daran gebundenen Atome oder Moleküle miteinander verbunden oder vernetzt sind, entfernt werden, um eine Ultrafeinstruktur zu bilden, die nur aus kontinuierlichen Atomen oder Molekülen besteht.

4. Ein wie in Anspruch 1 beanspruchtes Verfahren, welches umfaßt, daß die zusammengefügten Flagelline in einem Magnetfeld in einer Richtung ausgerichtet werden.

5. Ein Verfahren gemäß irgendeinem der vorhergehenden Ansprüche, bei dem das bakterielle Flagellin eine Untereinheit eines bakteriellen Flagellums ist.

6. Ein wie in Anspruch 5 beanspruchtes Verfahren, bei dem das Flagellin von einer Salmonella-Art abstammt.

7. Ein wie in irgendeinem der vorhergehenden Ansprüche beanspruchtes Verfahren, bei dem die an die Amino- oder Carboxylenden gebundenen Atome oder Moleküle ein elektrisch leitendes Filament bilden.

8. Eine Ultrafeinstruktur, die durch das Verfahren nach irgendeinem der Ansprüche 1 bis 7 erhältlich ist.

9. Flagellin-Moleküle mit wenigstens an das Aminoende oder das Carboxylende gebundenen Atomen oder Molekülen, so daß beim Zusammenfügen eine flagellinartige Struktur gebildet wird.

10. Eine Gruppe von flagellinartigen Strukturen, die wie in Anspruch 9 beanspruchte Flagellin-Moleküle enthalten.

## Revendications

1. Procédé de formation d'une structure ultrafine, comprenant la liaison d'atomes ou de molécules à au moins l'extrémité aminée ou l'extrémité carboxylée d'une ou plusieurs flagellines, et l'assemblage d'un grand nombre desdites flagellines pour former ladite structure.

2. Procédé selon la revendication 1, dans lequel les flagellines assemblées sont chauffées ou irradiées pour y lier ou réticuler entre eux, les atomes ou les molécules.

3. Procédé selon la revendication 2, dans lequel les flagellines sont éliminées des flagellines assemblées dans lesquelles les atomes ou les molécules qui y sont liés, sont liés ou réticulés entre eux pour former une structure ultrafine consistant seulement en atomes ou en molécules continus.

4. Procédé selon la revendication 1, selon lequel on oriente les flagellines assemblées selon une direction dans un champ magnétique.

5. Procédé selon l'une quelconque des revendications précédentes, dans lequel la flagelline bactérienne est une sous-unité d'un flagelle bactérien.

6. Procédé selon la revendication 5, dans lequel la flagelline est dérivée d'une espèce de *salmonella.*

7. Procédé selon l'une quelconque des revendications précédentes, dans lequel les atomes ou les molécules liés à ladite extrémité aminée ou carboxylée, forment un filament électroconducteur.

8. Structure ultrafine pouvant être obtenue selon le procédé de l'une quelconque des revendications 1 à 7.

9. Molécules de flagelline comportant des atomes ou des molécules liés au moins à l'extrémité aminée ou à l'extrémité carboxylée, de sorte qu'une structure en flagelline est formée par assemblage.

10. Ensemble de structures analogues à la flagelline, comprenant des molécules de flagelline selon la revendication 9.
